# EUROPÄISCHE PATENTSCHRIFT

(11) **EP 0 786 662 B1**
(45) Veröffentlichungstag und Bekanntmachung des Hinweises auf die Patenterteilung: **22.09.2004**
(21) Anmeldenummer: 97100796.8
(22) Anmeldetag: 20.01.1997
(51) Int. Cl.: G01N 33/18, B01F 13/08, C12M 1/34

(54) **Verfahren zur Versorgung des Gasaustausches bei BSB-Messungen**
Method for regulating gas exchange in BOD-measurements
Procédé de régulation de l'échange de gaz en mesures BDO

(30) Priorität: 29.01.1996 DE 19603166
(43) Veröffentlichungstag der Anmeldung: 30.07.1997
(73) Patentinhaber: WTW Wissenschaftlich-Technische Werkstätten GmbH & Co. KG, 82362 Weilheim (DE)
(72) Erfinder: Rettig, Ulrich, 82407 Wielenbach (DE); Straub, Michael, 82399 Raisting (DE)
(74) Vertreter: Zipse + Habersack

(56) Entgegenhaltungen:
- DE-A- 2 010 515
- DE-A- 2 201 204
- US-A- 4 199 265
- US-A- 4 752 138

## Beschreibung

Die vorliegende Erfindung betrifft ein Verfahren zur Verbesserung des Gasaustauschs beim Bestimmen des biochemischen Sauerstoffbedarfs.

Die Messung des biochemischen Sauerstoffbedarfs ist ein Standardverfahren, um die Belastung von Abwässern zu messen. Ein derartiges Meßverfahren ist z.B. in der DIN 38409, Teil 51 spezifiziert. Die zu messende Abwasserprobe wird hierfür in einen Behälter, zumeist eine Glasflasche, gegeben. In dem Behälter ist ein Rührorgan beweglich angeordnet, das durch ein außerhalb des Behälters erzeugtes magnetisches Feld bewegbar ist. Das Rührorgan ist in der Regel als Magnetstäbchen ausgebildet, welches mit einer Kunststoffhülle umgeben ist, um das Magnetstäbchen gegen die Abwasserprobe zu schützen und um eine wechselseitige chemische Beeinflussung zu vermeiden. Durch das Rührorgan wird die Probe in Bewegung gehalten, um eine bessere Durchmischung der Probe und somit eine Beschleunigung der physikalisch-chemischen Reaktionen und des Gasaustauschs zu erreichen.

Aus der DE-A 44 23 848 ist eine derartige Vorrichtung bekannt. Der Behälter besteht dort aus einer Glasflasche, auf deren Oberseite ein elektronischer Meßkopf zur Bestimmung des biochemischen Sauerstoffbedarfs angeordnet ist. Üblicherweise sind mehrere derartige Glasflaschen neben und hintereinander auf einer gemeinsamen Rührplattform angeordnet. Ein besonderes Problem, das sich bei derartigen Anordnungen ergibt, besteht darin, dass sich die magnetischen Rührorgane der bei kompakten Rührplattformen dicht nebeneinander stehenden Flaschen gegenseitig beeinflussen können, so dass die sich gegenseitig anziehenden Rührorgane durch das Drehfeld der Rührplattform überhaupt nicht in Drehung versetzt werden. Ein weiteres Problem derartiger Vorrichtungen besteht darin, dass die Durchmischung der Probe in den Glasflaschen nicht immer zufriedenstellend ist. Ein weiterer Nachteil herkömmlicher Rührplattformen besteht darin, dass es beim Aufsetzen einer Probe auf eine Rührplattform vorkommen kann, dass das Rührorgan aufgrund des größeren Widerstandes der Abwasserprobe mit dem Drehfeld nicht synchronisiert wird.

Die DE-A 220124 zeigt ein Gerät zur Bestimmung des biochemischen Sauerstoffbedarfs beim Abbau von organischen Stoffen im Abwasser. Es enthält eine Messvorrichtung zur Bestimmung des Wasserstoffbedarfs und einen mit einem Magneten versehenen Behälter. Die Drehfrequenz des Magneten wird dabei mittels einer Antriebsanordnung gesteuert. Dieses Dokument lehrt ausführlich wie ein auf dem Boden des Probenbehälters ruhender und frei drehbarer Rührer anzuregen ist, um eine bestimmte Rühr- oder Drehfrequenz zu erreichen. Nachdem die gewünschte Drehfrequenz erreicht ist, wird diese in dem Gerät konstant gehalten.

Die DE-A 20 10 515 und die US 4,199,265 beschreiben ein Magnetrührgerät zum Rühren von flüssigen Medien. Eine Verbesserung der Mischwirkung wird gemäß dieser Druckschrift dadurch erzielt, dass der Dreh- bzw. Umlaufsinn des umlaufenden Magnetfeldes in bestimmten Zeitabständen umkehrt.

Die US 4,752,138 beschreibt eine Magnetrühranordnung nach dem Stand der Technik. In Spalte 4, Zeilen 49 bis 68, dieser Druckschrift wird beschrieben die Steigerung der Anfahrrührfrequenz während der Anfahrphase zu variieren.

Es ist daher Ziel der Erfindung, ein Verfahren zur Verbesserung des Gasaustauschs beim Bestimmen des biochemischen Sauerstoffbedarfs zu schaffen, das eine gute Durchmischung der zu messenden Probe auch bei Rührplattformen mit dich nebeneinander angeordneten Behältern erlaubt und einen unproblematischen automatischen Anlauf des Rührorgans beim Aufsetzen von Behältern auf die laufende Rührplattform gewährleistet.

Dieses Ziel wird durch ein Verfahren gemäß Anspruch 1 gelöst. Vorteilhafte Weiterbildungen der Erfindung sind Gegenstand der Unteransprüche.

Erfindungsgemäß wird durch das Verfahren der Erfindung ein Drehfeld erzeugt, dessen Drehfrequenz, vorzugsweise periodisch, bei gleichbleibendem Drehsinn variiert. Dies hat mehrere Vorteile. Zum einen ist es dem Rührorgan eines neu auf die Rührplattform aufgestellten Behälters möglich, in der Phase der Frequenzabsenkung mit dem Drehfeld der Rührvorrichtung zu synchronisieren. Die Variation der Drehfrequenz des Drehfeldes hat also derart zu erfolgen, dass die Frequenz des Drehfeldes, möglichst periodisch, abgesenkt wird. In dieser Zeit niedriger Frequenz, die bis zum Stop des Drehfeldes gehen kann, ist es für das Rührorgan möglich, direkt mit seinen beiden entfernt liegenden magnetischen Polen von dem sehr langsam drehenden oder stehenden Drehfeld erfaßt zu werden. Hierbei werden auch Rührorgane eng benachbarter Behälter voneinander getrennt und in dem Drehfeld ausgerichtet.

Ein weiterer Vorteil der Frequenzvariation besteht darin, dass die Durchmischung der Probe stark verbessert wird, denn die Probe hat immer das Bestreben, mit dem Rührorgan mitzudrehen. Es wird somit bei herkömmlichen Rührapparaturen mit konstant drehendem Rührorgan letztendlich ein rotierender Zylinder der Abwasserprobe erzeugt, der die Durchmischung und den Gasaustausch wieder stärker unterbindet. Durch die Frequenzvariation hingegen werden ständig Verwirbelungen zwischen dem Rührorgan und der Probe erzeugt, da das Rührorgan durch die Frequenzvariation zeitweise der sich drehenden Abwassersäule voreilt und zeitweise diese abbremst. Dieser Effekt läßt sich verstärken, wenn die Frequenzvariation einen Stop des Drehfeldes und sogar eine Richtungsumkehr des Drehfeldes beinhaltet. So wird die Ausbildung einer sich mit konstanter Geschwindigkeit rotierenden Abwassersäule in dem Behälter verhindert.

Die Periode eines Variationszyklus, d.h. der zeitliche Abstand von einer Frequenzvariation zur nächsten beträgt vorzugsweise zwischen 5 und 180 Sekunden. Wird die Variationszeit zu kurz gewählt, so hat das Rührorgan nicht genügend Zeit, mit dem Drehfeld zu synchronisieren. Wird die Periode zu lang gewählt, so tritt in den Zeiten verringerter Drehfrequenz eine Verringerung des Mischeffektes auf, der wiederum nicht erwünscht ist. Vorteilhaft ist eine Periode zwischen 10 und 30 Sekunden. Der Zyklus für die Veränderung kann konstant sein oder gesteuert bzw. durch einen Zufallsgenerator beliebig variiert werden. Die Frequenzvariation kann in diskreten Zeitspannen zwischen Zeitabschnitten konstanter Frequenz vorgenommen werden, wodurch man einerseits eine leicht zu berechnende Rührenergie in die Probe einbringt und andererseits in den vorzugsweise kürzeren Phasen der Frequenzvariation einen definierten Zeitpunkt für den Anlauf eines Rührorgans in einem später auf die Rührplattform aufgesetzten Behälter erzielt. Die Frequenzvariation kann jedoch auch kontinuierlich über den gesamten Betrieb der Rührplattform, z.B. in Form einer Sinus- oder Dreickkurve, erfolgen, in welchem Fall die Mischwirkung über den gesamten Betrieb aufgrund der ständigen Veränderung der Drehzahl des Rührorgans verbessert wird.

Die Frequenzvariation kann derart durchgeführt werden, dass bei der Absenkung der Drehfrequenz das Drehfeld gestoppt wird. Dann hat das Rührorgan genug Zeit, mit seinen voneinander abweisenden Polen sich über den entsprechenden Polen des gestoppten magnetischen Drehfeldes anzuordnen. Die durch die Frequenzvariation verbesserte Synchronisation des Rührorgans mit dem Drehfeld wird somit bei einem Stop des Drehfeldes abermals verbessert.

Durch die Frequenzvariation kann ein Rührorgan mit dem Drehfeld synchronisiert werden, auch wenn die Flasche einen gewölbten Flaschenboden aufweist. Dies führte bislang dazu, dass sich das als längliches Magnetstäbchen ausgebildete Rührorgan in der Regel seitlich am Flaschenrand anlagerte. Gerade diese Stellung führt jedoch bei dicht nebeneinander auf einer Rührplattform stehenden Flaschen dazu, dass sich die Rührorgane benachbarter Flaschen anziehen. Hier können durch die Frequenzvariation und gegebenenfalls den periodischen Stop und/oder Richtungsumkehr des Drehfeldes die sich gegenseitig anziehenden Rührorgane benachbarter Flaschen getrennt werden.

Um den Mischeffekt bei der Frequenzvariation zu verbessern, kann die Frequenzanhebung und -absenkung in diskreten Schritten erfolgen. Dies hat den Effekt, dass die sich drehende Probensäule bei einer diskreten Frequenzänderung im Bereich des Rührstäbchens stärker verwirbelt wird als bei einer kontinuierlichen Änderung der Frequenz.

Die diskrete Frequenzvariation des Drehfeldes der Rührvorrichtung kann vorzugsweise mittels eines Schrittmotors zum Drehen eines Magneten oder Elektromagneten realisiert werden, oder durch ein mit Spulen elektromagnetisch erzeugtes Drehfeld unter Zugrundelegung einer entsprechenden Steuerung, die die Spulen in entsprechender Weise ansteuert.

Ein derartiges Gerät mit einer Rührplattform zur Aufnahme mehrerer Behälter kann also sehr kompakt und dicht gebaut werden, d.h. Behälter können dichter als bisher nebeneinander angeordnet werden, ohne dass die Rührfunktion der Rührorgane der nebeneinander liegenden Behälter sich gegenseitig in ihrer Grundfunktion beeinflussen.

Vorzugsweise ist die Steuerung für die Rührvorrichtung durch einen Mikroprozessor gebildet. So kann z.B. über die interne Uhr des Mikroprozessors die zeitliche Steuerung der Drehfrequenz realisiert werden. Es kann nicht nur die Drehzahl und/oder die Drehrichtung variiert werden, sondern auch die Feldstärke des Magnetfeldes im Falle einer elektromagnetischen Ansteuerung. Vorzugswiese wird die Intensität und Kurvenform des magnetischen Feldes synchron mit der Frequenzvariation verändert. Dies hat den Vorteil, dass bedarfsgerecht für den jeweiligen zeitlichen Abschnitt innerhalb einer Periode die Beeinflussung des Rührorgans erfolgt und sich dadurch geringe E-nergieumsätze erzielen lassen, was in erwünschter Weise zu einer geringen Probenerwärmung führt. Hierbei können den einzelnen Abschnitten einer Periode jeweils eine Dauer, eine Frequenz, eine beliebige Kurvenform und eine Intensität des magnetischen Feldes zugeordnet werden.

In einer technisch einfach zu realisiernden Ausführungsform wird das Drehfeld mit einer definierten Taktfrequenz gedreht, und eine Frequenzvariation wird dadurch erzielt, dass die Schrittweite von groß (Vollschritt) bis klein (Viertelschritt) variiert wird. Vorzugsweise wird beim Zentrieren des Rührorgans vom Behälterrand zur Behältermitte in einer Phase geringerer Drehfrequenz und beim Anlauf des Rührorgans ( Vollschrittverfahren) ein Feld mit maximaler Feldstärke, beim Beschleunigen und Verzögern des Rührorgans (Halb- oder Viertelschrittverfahren) ein Feld mit mittlerer Intensität und während des Gleichlaufs des Rührorgans, d.h. in einer Phase konstanter Frequenz (Viertelschrittverfahren) ein Drehfeld mit minimaler Intensität benutzt. Durch die Erhöhung der Feldstärke in einer Phase verminderter Frequenz wird eine sichere Zentrierung und Synchronisation des Feldes erzielt, während durch eine Minimierung der Feldstärke in Phasen konstanter Frequenz der Einfluß des Feldes auf die Probe weitgehend verringert wird.

Durch die verbesserte Anbindung der Rührorgane an das elektromagnetische Feld aufgrund der Frequenzvariation lassen sich die Behälter dichter nebeneinander plazieren als es bisher möglich war, und es lassen sich Flaschen mit gewölbtem Boden verwenden.

Ein weiterer Vorteil des erfindungsgemäßen Verfahrens besteht darin, dass durch die Frequenzvariation des Drehfeldes die magnetische Feldstärke des Feldes verringert werden kann, so dass das mit dem erfindungsgemäßen Verfahren arbeitende Gerät zum einen mit einer geringeren elektrischen Leistung betrieben werden kann, und zum anderen das Probengut einem schwächeren Magnetfeld und einer geringeren Erwärmung aufgrund der eingebrachten Rührleistung unterworfen ist.

Bei Verwendung einer Rührplattform mit nebeneinander angeordneten Stellflächen für die Behälter ist es vorteilhaft, wenn die Stellflächen in einer Sicherheitswanne angeordnet sind, so dass bei Zerstörung einer Flasche das Probengut in der Sicherheitswanne verbleibt.

Für die Erfindung ist es lediglich wichtig, dass die Frequenz des Drehfeldes variiert wird. Wie die Variation erfolgt ist zweitrangig. Diese Änderung kann z.B. periodisch oder zufallsgesteuert erfolgen. In der Praxis wird eine periodische Variation der Frequenz wegen der definierteren Mischungsparameter vorzuziehen sein.

Vorzugsweise enthält ein nach dem erfindungsgemäßen Verfahren arbeitendes Gerät nur eine Steuerung für alle Rührvorrichtungen zur Erzeugung der Drehfelder, die unter der Rührplattform angeordnet sind. So können alle Rührvorrichtungen in gleicher Weise mit einem variierenden Drehfeld angesteuert werden, ohne dass dies negative Auswirkungen im Sinne einer wechselseitigen Beeinflussung nebeneinander angeordneter Rühranordnungen hätte. Das Rührgerät und das entsprechende Verfahren eignen sich nicht nur zur BSB-Messung, sondern auch für andere Verfahren, in denen der Gasaustausch bzw. das Rühren eine wichtige Rolle spielen.

## Patentansprüche

1. Verfahren zur Verbesserung des Gasaustauschs beim Bestimmen des biochemischen Sauerstoffbedarfs einer in einem Behälter angeordneten Probe, die durch ein Rührorgan in Bewegung versetzt wird, welches Rührorgan durch eine außerhalb des Behälters angeordnete Rührvorrichtung mittels eines magnetischen Drehfeldes betätigt wird,
**dadurch gekennzeichnet, dass** die Drehfrequenz des Drehfeldes während des eigentlichen Mischvorgangs der Rührvorrichtung bei einem gleichbleibenden Drehsinn zyklisch mit konstantem oder variabel steuerbaren Zyklus variiert wird.

2. Verfahren nach Anspruch 1,
**dadurch gekennzeichnet,**
**dass** die Drehfrequenz periodisch variiert wird.

3. Verfahren nach Anspruch 1 oder 2,
**dadurch gekennzeichnet,**
**dass** die Frequenz in diskreten Schritten variiert wird.

4. Verfahren nach einem der vorherigen Ansprüche,
**dadurch gekennzeichnet,**
**dass** die Frequenzvariation einen Stop des Drehfeldes beinhaltet.

5. Verfahren nach einem der vorherigen Ansprüche,
**dadurch gekennzeichnet,**
**dass** die Feldstärke des Drehfeldes in Synchronisation mit der Frequenz des Feldes variiert wird.

## Claims

1. A method for improving the gas exchange in determining the biochemical oxygen demand (BOD) of a sample arranged in a vessel, said sample being set in motion by an agitator member actuated by an agitator outside of the vessel by means of a magnetic rotary field,
**characterized in that** during the actual mixing action of the agitator the frequency of rotation of the rotary field is varied in a cycle controllable to be constant or variable with no change in the sense of rotation.

2. The method as set forth in claim 1,
**characterized in that**
the frequency of rotation is varied periodically.

3. The method as set forth in claim 1 or 2,
**characterized in that**
the frequency is varied in discrete steps.

4. The method as set forth in any of the preceding claims,
**characterized in that**
the frequency variation includes a rotary field stop.

5. The method as set forth in any of the preceding claims,
**characterized in that**
the field strength of the rotary field is varied in synchronism with the frequency of the field.

## Revendications

1. Procédé pour améliorer l'échange de gaz lors de la détermination du besoin biochimique d'oxygène d'un échantillon disposé dans un récipient, lequel échantillon est mis en mouvement par un organe d'agitation, lequel est actionné par un dispositif d'agitation , disposé à l'extérieur du récipient, au moyen d'un champ tournant magnétique, **caractérisé en ce que** la fréquence de rotation du champ tournant peut être variée de façon cyclique avec un cycle constant ou susceptible d'être variable pendant le processus de mélange proprement dit du dispositif d'agitation par un sens de rotation constant.

2. Procédé selon la revendication 1, **caractérisé en ce que** la fréquence de rotation varie de façon périodique.

3. Procédé selon la revendication 1 ou 2, **caractérisé en ce que** la fréquence peut être variée selon des pas discrets.

4. Procédé selon une des revendications précédentes, **caractérisé en ce que** la variation de fréquence comprend un arrêt du champ tournant.

5. Procédé selon une des revendications précédentes, **caractérisé en ce que** l'intensité du champ tournant varie en synchronisation avec la fréquence du champ.
